# EUROPEAN PATENT SPECIFICATION

(11) **EP 4 669 392 B1**
(45) Date of publication and mention of the grant of the patent: **25.11.2026**
(21) Application number: 24714307.6
(22) Date of filing: 20.02.2024
(51) Int. Cl.: A61M 5/315

(54) **ELECTROMECHANICAL ARCHITECTURE FOR CONNECTED PREFILLED DISPOSABLE SYRINGES**
ELEKTROMECHANISCHE ARCHITEKTUR FÜR VERBUNDENE VORGEFÜLLTE EINWEGSPRITZEN
ARCHITECTURE ÉLECTROMÉCANIQUE POUR SERINGUES JETABLES PRÉ-REMPLIES CONNECTÉES

(30) Priority: 21.02.2023 US 202363447237 P
(43) Date of publication of application: 31.12.2025
(73) Proprietor: Flex Ltd., 486123 Singapore (SG)
(72) Inventor: DE ANGELI, Marco, 24030 BARZANA (IT)
(74) Representative: FRKelly
(86) International application number: PCT/US2024/016480
(87) International publication number: WO 2024/177976

(56) References cited:
- US-A- 5 720 733
- US-A1- 2017 274 149
- US-A1- 2021 046 247
- US-A1- 2021 146 062

## Description

### TECHNICAL FIELD

The present disclosure is directed to disposable syringes, and more particularly it is directed to prefilled disposable syringes with an electromechanical architecture for data collection and transfer.

### BACKGROUND OF THE INVENTION

As is readily known, a syringe is a pumping device generally including a plunger disposed tightly within a cylindrical tube or barrel, and syringes are generally used in the medical industry to inject and dispense a fluid, such as liquid medication. Recently, there is a desire to obtain more data related to medical procedures to improve clinical trials and the overall health of patients. Therefore, smart syringes have been developed which are utilized to collect injection information, provide more refined insights, and perform better statistical analysis of medical trial outcomes. For example, traditional smart syringes can be configured to record one or more of the following: time of injection, duration of injection, dose administered, drug name, expiry date, and other similar data. An issue arises when introducing the smart syringe technology into disposable syringes due to the higher costs associated with smart syringes and the disposable nature of disposable syringes, resulting in an expensive disposable product.

Therefore, there is a need for a cost effective disposable syringe that utilizes traditional smart syringe technology, as well as includes further capabilities to collect data that has not previously been collected using smart syringe technology.

US2021/046247 relates to drug delivery systems and methods of use thereof for recording administration of a drug dose to a subject. Aspects include a syringe stopper rod comprising a sensor component that is configured to detect a delivery signature, and to transmit a report comprising a drug dose completion signal to a data management component, e.g., a mobile computing device. US2017/274149 relates to apparatus and systems for enabling and monitoring injection of medicinal fluids into a subject. The apparatus includes a housing for a prefilled syringe, the housing incorporating means for generating and sending signals as parts of the syringe cooperate to deliver the fluids. The signals may be transmitted from a transmitter on the housing as radio waves to a receiver, which may be conveniently located on a spaced device, such as a mobile phone. US5720733 relates to an apparatus for electrically determining and recording the dose of an agent delivered with a syringe of the type having a barrel for holding the agent and a plunger for expelling the agent. The syringe includes a capacitive element having first and second conducting surfaces arranged such that the capacitance of the capacitive element varies in dependence upon the dose of agent contained in the barrel. An input terminal and an output terminal located on the outside of the syringe are electrically connected to the first and second conducting surfaces, respectively. The apparatus produces a voltage difference across the terminals, thereby charging the capacitive element. A capacitance meter measures the capacitance and a microprocessor calculates the dose from the measured capacitance. The calculated dose is recorded by a digital memory unit. US2021/146062 relates to a position tracking system which includes a member extending along a lengthwise axis. Multiple position tracking elements (such as conductive strips) are fabricated to be exposed on a surface of the member. The multiple position tracking elements form a sequence along the lengthwise axis and are spaced apart from each other. The position tracking system further includes a probe. The probe monitors for presences and absence of the position tracking elements disposed in the sequence as the member and corresponding sequence of position tracking elements moves with respect to the probe. Based on the detected presence and absence of position tracking elements, a monitor resource of the position tracking system monitors parameters such as: i) a position of the member along the axis, ii) a rate of movement of the member along the axis over time, iii) a direction of movement of the member along the axis, etc.

### SUMMARY OF THE INVENTION

According to one aspect, a syringe (i.e., a "smart syringe") is disclosed according to the present disclosure. The syringe can include a barrel including a barrel flange positioned at an axial end of the barrel. A plunger can be axially translatable within the barrel along a central axis of the plunger, and the plunger can include a first body portion coupled to a second body portion. A printed circuit board can be coupled to and positioned between the first body portion and the second body portion. The printed circuit board can include a ground contact disposed on a first lateral side of the printed circuit, a plurality of contact pads disposed on a second lateral side of the printed circuit board, and a microcontroller coupled to a surface of the printed circuit board. A conductive ring can be disposed about an outer circumference of the plunger, the conductive ring can include sliding contacts configured to engage the ground contact and the plurality of contact pads of the printed circuit board. The microcontroller can be configured to detect a change in conductivity to identify a position of the plunger relative to the barrel.

In one aspect, the first body portion and the second body portion are coupled together through a snap-fit connection.

In one aspect, the first body portion and the second body portion are a first half portion and a second half portion of the plunger, respectively.

In one aspect, the second body portion includes a pin positioned on a radially inner surface of the second body portion, relative to the central axis of the plunger.

In one aspect, the printed circuit board includes an aperture extending through the printed circuit board, the aperture being configured to accept the pin of the second body portion for positioning of the printed circuit board relative to the second body portion.

In one aspect, the printed circuit board includes a battery connector disposed at an end of the printed circuit board, the battery connector being configured for accepting and securing a battery of the syringe.

In one aspect, the battery is a coin cell battery.

In one aspect, the ground contact of the printed circuit board is a continuous ground contact that extends from a first end of the printed circuit board to a second end of the printed circuit board.

In one aspect, the plurality of contact pads are a discontinuous group of individual contact pads that extend from a first end of the printed circuit board to a second end of the printed circuit board.

In one aspect, the discontinuous group of individual contact pads includes a nonconductive space positioned between each adjacent contact pad of the plurality of contact pads.

In one aspect, the microcontroller is a Bluetooth low energy chip that is configured to wirelessly transmit data to a device remote from the syringe.

In one aspect, the surface of the printed circuit board the microcontroller is coupled to is perpendicular to the first lateral side and the second lateral side of the printed circuit board.

In one aspect, the first lateral side of the printed circuit board is parallel with the second lateral side of the printed circuit board.

In one aspect, the syringe is a pre-filled disposable syringe.

In one aspect, the conductive ring includes a circular shaped conductive ring body and a first sliding contact and a second sliding contact each extending axially away from the conductive ring body.

In one aspect, the first sliding contact is configured to engage the ground contact of the printed circuit board, and the second sliding contact is configured to engage the plurality of contact pads of the printed circuit board.

In one aspect, the conductive ring includes an inner diameter greater than an outer diameter of a portion of the plunger configured to axially translate into the barrel.

In one aspect, the conductive ring is axially translatable along an outer surface of the plunger during an axial translation of the plunger into the barrel.

In one aspect, during the axial translation of the plunger into the barrel the conductive ring contacts the barrel flange, preventing axial translation of the conductive ring relative to the barrel.

In one aspect, before the axial translation of the plunger into the barrel, the conductive ring is axially offset from the barrel flange.

### BRIEF DESCRIPTION OF THE DRAWINGS

The foregoing Summary as well as the following Detailed Description will be best understood when read in conjunction with the appended drawings, which illustrate a preferred embodiment of the disclosure. In the drawings:
FIG. 1 is a perspective view of a syringe according to the present disclosure.
FIG. 2 is a side view of a plunger of the syringe of FIG. 1.
FIG. 3 is an exploded perspective view of the plunger of FIG. 2.
FIG. 4 is a perspective view of a printed circuit board of the plunger of FIG. 2.
FIG. 5 is a perspective view of a conductive ring of the syringe of FIG. 1.
FIG. 6A is a perspective view of the syringe of FIG. 1 in a first position.
FIG. 6B is a perspective view of the syringe of FIG. 1 in a second position.
FIG. 6C is a perspective view of the syringe of FIG. 1 in a third position.

### DETAILED DESCRIPTION OF THE PREFERRED EMBODIMENTS

Certain terminology is used in the following description for convenience only and is not limiting. The words "front", "rear", "upper", and "lower" designate directions in the drawings to which reference is made. The words "inwardly" and "outwardly" refer to directions towards and away from parts referenced in the drawings. "Axially" refers to a direction along the axis of a shaft, pin, tube, barrel, rod, or the like. A reference to a list of items that are cited as "at least one of a, b, or c" (where a, b, and c represent the items being listed) means any single one of the items a, b, or c, or combinations thereof are included. The terminology includes the words specifically noted above, derivatives thereof, and words of similar import.

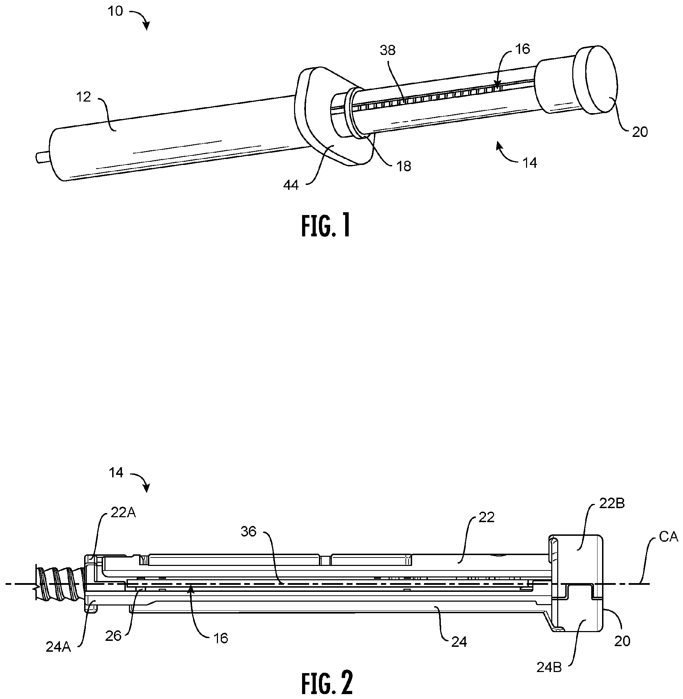

FIG. 1 is a perspective view of a syringe 10 according to the present disclosure. The syringe 10 is a pumping device which is generally used in the medical industry to inject and dispense a fluid such as liquid medication, among other options not specifically listed. In some examples, the syringe 10 can be a pre-filled disposable syringe, such that a user receives the syringe pre-filled with a fluid, injects the fluid, and then disposes of (throws away) the pre-filled disposable syringe. In other examples, the syringe 10 may not be a pre-filled or disposable syringe.

The syringe 10 can include a barrel 12, a plunger 14, a printed circuit board 16 within the plunger 14, a conductive ring 18, and a needle (not shown). The barrel 12 is the body of the syringe 10 that houses the plunger 14 and the fluid to be dispensed by the syringe 10. In some examples, as illustrated, the barrel 12 can have a generally elongated cylindrical shape. In other examples, the barrel 12 can have a shape other than an elongated cylindrical shape. Disposed at the end of the barrel 12, the needle can be coupled to the barrel 12. As is known, the needle is configured to be inserted into the user when dispensing the fluid or medication from within the syringe 10. The plunger 14 is disposed tightly within the barrel 12. Specifically, the plunger 14 is axially aligned with the barrel 12, and the plunger 14 is axially translatable within the barrel 12 along a central axis CA of the plunger 14. When pressed and/or squeezed, an end of the plunger 14 (not illustrated in FIG. 1) is configured to force the fluid within the barrel 12 out through the needle (not shown). The plunger 14 includes a plunger end 20 extending outside the barrel 12, which a user presses or squeezes to force the plunger 14 to axially translate into the barrel 12 to force the fluid within the barrel 12 out through the needle, as is readily known.

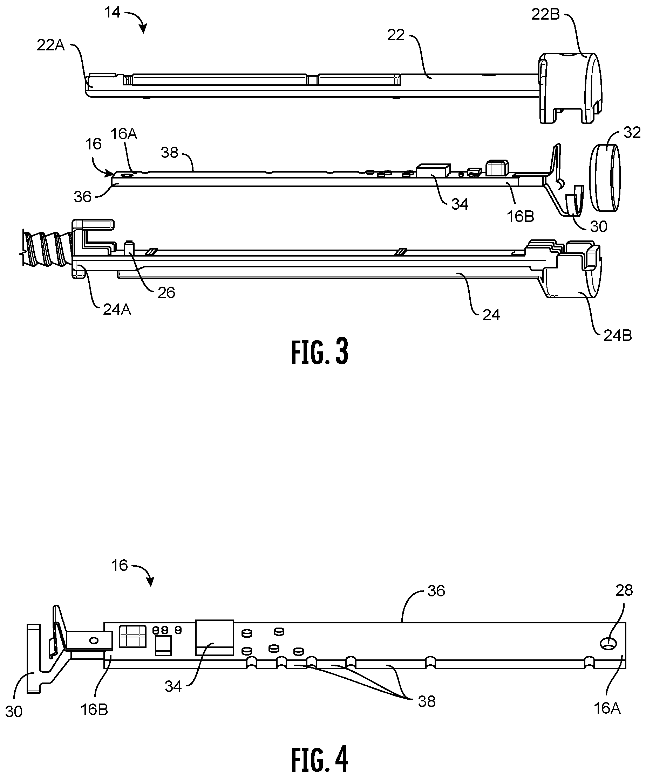

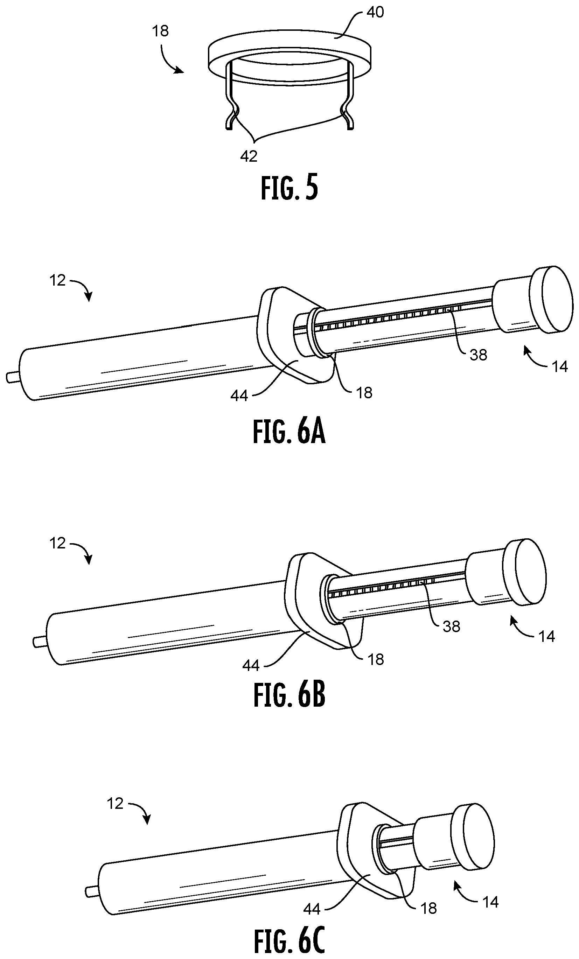

FIG. 2 is a side view of the plunger 14 of the syringe 10 illustrated in FIG. 1. FIG. 3 is an exploded perspective view of the plunger 14. FIG. 4 is a perspective view of the printed circuit board 16 of the plunger illustrated in FIG. 3. FIG. 5 is a perspective view of the conductive ring 18 of the syringe 10 illustrated in FIG. 1. FIGS. 2-5 will be discussed together. As illustrated, the plunger 14 can include a first body portion 22 and a second body portion 24 with the printed circuit board 16 positioned between the first body portion 22 and the second body portion 24. Specifically, the first body portion 22 and the second body portion 24 can each be elongated axially extending halves of the plunger 14 that are coupled together to form the overall plunger 14. Additionally, the printed circuit board 16 can be disposed centrally between the first body portion 22 and the second body portion 24, such that the printed circuit board 16 is positioned radially between outer surfaces of the plunger 14 with respect to the central axis CA of the plunger 14. In other words, the printed circuit board 16 is positioned generally aligned with the central axis CA of the plunger 14, between the first body portion 22 and the second body portion 24, discussed further below.

The first body portion 22 includes a first end 22A and a second end 22B that are positioned at opposite distal ends from each other. Likewise, the second body portion 24 includes a first end 24A and a second end 24B that are positioned at opposite distal ends from each other. The shape and size of the first end 22A of the first body portion 22 is configured to be a mating profile with the first end 24A of the second body portion 24, such that the first ends 22A, 24A can be coupled together. Similarly, the shape and size of the second end 22B of the first body portion 22 is configured to be a mating profile with the second end 24B of the second body portion 24, such that the second ends 22B, 24B can be coupled together to form the overall plunger 14. In some examples, as illustrated, the first end 22A of the first body portion 22 can include a straight portion and the first end 24A of the second body portion 24 can include a hook portion. As such, the first ends 22A, 24A can be coupled together such that the straight portion of the first end 22A is held within the hook portion of the first end 24A. Additionally, the second body portion 24 can include a pin 26 that can be configured to engage with an aperture (not shown) of the first body portion 22 in an interference or friction fit to aid in positioning and coupling the components together. Further, the second ends 22B, 24B can each include mating clip features that are configured to snap-clip together to secure the second ends 22B, 24B together. It is to be understood that the illustrated and disclosed coupling technique is only an example and the first body portion 22 and the second body portion 24 can be coupled together using any other technique that will secure the first body portion 22 and the second body portion 24 together during use of the syringe 10.

Referring again to FIGS. 2-4, the printed circuit board 16 includes a shape that generally corresponds to a shape of the first body portion 22 and the second body portion 24. Specifically, the printed circuit board 16 can generally have a thin and long rectangular shape, such that the printed circuit board 16 fits between the first body portion 22 and the second body portion 24. The printed circuit board 16 can include an aperture 28 extending through a first end 16A of the printed circuit board 16. The aperture 28 can be sized and shaped to accept the pin 26 of the second body portion 24, and the aperture 28 and pin 26 connection is configured to properly position and secure the printed circuit board 16 relative to the first body portion 22 and the second body portion 24. Therefore, to install the printed circuit board 16 to the plunger 14, the first body portion 22 and the second body portion 24 are initially separated (see. FIG. 3) and then the printed circuit board 16 is inserted onto the second body portion 24, such that the pin 26 of the second body portion 24 is inserted into the aperture 28 of the printed circuit board 16. The connection between the aperture 28 of the printed circuit board 16 and the pin 26 of the second body portion 24 properly aligns the printed circuit board 16 on the second body portion 24. Then the first body portion 22 is coupled to the second body portion 24 by coupling the respective first ends 22A, 24A and second ends 22B, 24B together, as previously discussed. Therefore, as illustrated best in FIG. 2, after the printed circuit board 16 is coupled within the plunger 14, the printed circuit board 16 is coupled (i.e., sandwiched) between the first body portion 22 and the second body portion 24 of the plunger 14.

Referring now to FIG. 4, the printed circuit board 16 includes the aperture 28 positioned adjacent the first end 16A of the printed circuit board 16. Further, the printed circuit board 16 includes a battery connector 30 positioned at a second end 16B of the printed circuit board 16, with the second end 16B being an opposite end of the printed circuit board 16 as the first end 16A. The battery connector 30 is an electrical connection feature that is configured to accept a battery 32 (see FIG. 3), which battery 32 provides electrical energy to power and operate the electrical components of the printed circuit board 16. In some examples, as illustrated, the battery 32 can be a coin or button cell battery, and the battery connector 30 can be sized and shaped to secure the battery 32 to the printed circuit board 16. Additionally, the battery connector 30 and the battery 32 can be sized and shaped to fit within an interior of each of the second ends 22B, 24B of the first body portion 22 and the second body portion 24, which protects and seals the battery 32 from encountering fluid or other debris during use. In some examples, as illustrated, a widthwise direction or diameter of the battery 32 can be oriented approximately perpendicular to a direction extending from the first end 16A to the second end 16B of the printed circuit board 16.

The printed circuit board 16 can further include a Bluetooth Low Energy (BLE) chip 34, a ground contact 36, and a plurality of contact pads 38. The BLE chip 34 can also be referred to as a BLE microcontroller or microcontroller, the BLE chip 34 is electrically coupled to the printed circuit board 16, and the BLE chip 34 is configured to receive electrical energy from the battery 32. The BLE chip 34 is configured to read a digital input change of the printed circuit board 16, and then a newly computed status of the syringe 10 can be sent by the BLE chip 34 to a smartphone or other device, which can then store and/or analyze the data received from the BLE chip 34 of the syringe 10, discussed further below. In some examples, the BLE chip 34 can have a transmission range of 10 meters or greater to transfer data to a connected device. The ground contact 36 is positioned and extends along a first lateral side of the printed circuit board 16 from the first end 16A to the second end 16B of the printed circuit board 16. Further, the ground contact 36 is a continuous ground contact 36 that extends fully along a first lateral side of the printed circuit board 16 from the first end 16A to the second end 16B of the printed circuit board 16. The ground contact 36 can be produced from a conductive material, such as for example a copper material.

The plurality of contact pads 38 are positioned and extend along a second lateral side of the printed circuit board 16, opposite the first lateral side, from the first end 16A to the second end 16B of the printed circuit board 16. Further, the plurality of contact pads 38 are a discontinuous group of individual contact pads 38 that are each separated by small nonconductive gaps or distances between each individual contact pad 38. In some examples, as illustrated in FIG. 4, the relative size and shape of each individual contact pad 38 may differ to differentiate between each individual contact pad 38 and the electrical signal produced by each individual contact pad 38. In other examples, as illustrated in FIG. 1, the relative size and shape of each individual contact pad 38 may be the same. In such an example, a different technique may be used to identify each individual contact pad 38, discussed further below. The printed circuit board 16 can be conformal coated in a thin polymeric film that is applied to the printed circuit board 16 in order to protect the printed circuit board 16 and its components from the environment and corrosion. Specifically, the printed circuit board 16 can be conformal coated to improve the resilience to water or other fluid ingress, leaving only the ground contact 36 and the plurality of contact pads 38 exposed for a conductive connection with the conductive ring 18, discussed below.

Referring now to FIG. 5, which is a perspective view of the conductive ring 18 of the syringe 10 illustrated in FIG. 1. The conductive ring 18 includes a circular ring shaped conductive ring body 40 that is constructed from a conductive material, such as for example a copper material. The conductive ring body 40 is shaped to fit around an outer circumference of the plunger 14. As such, the conductive ring body 40 has an inner diameter that is approximately equal to (and slightly larger than) an outer diameter of a least a portion of the plunger 14. Further, the conductive ring body 40 is configured to slide or translate axially along the outer circumference of the plunger 14 during the axial translation of the plunger 14 into the barrel 12. The conductive ring 18 also includes at least one sliding contact 42 coupled to and generally extending axially away from the conductive ring body 40. In the illustrated example, the conductive ring 18 includes two sliding contacts 42. In other examples, the conductive ring 18 can include more or less than two sliding contacts 42. The sliding contacts 42 are configured to engage and contact the ground contact 36 and the plurality of contact pads 38 when the conductive ring 18 is assembled to the plunger 14, discussed below.

FIG. 6A is a perspective view of the syringe 10 in a first position or orientation before an injection. FIG. 6B is a perspective view of the syringe 10 in a second position or orientation during an injection. FIG. 6C is a perspective view of the syringe 10 in a third position or orientation after completing an injection. FIGS. 6A-6C will be discussed together. As illustrated in FIGS. 6A-6C, the conductive ring 18 is coupled to the plunger 14 such that the conductive ring 18 is axially aligned with the central axis CA of the plunger 14. Further, as illustrated in FIG. 6A, the conductive ring 18 is coupled to the plunger 14 such that the conductive ring 18 is positioned near the first ends 22A, 24A, which are inserted into the barrel 12 of the syringe 10. Additionally, as illustrated in FIG. 6A, when the plunger 14 is inserted into the barrel 12 and the barrel 12 is filled with a fluid (e.g. a liquid medication), the conductive ring 18 is axially offset from a barrel flange 44 of the barrel 12. The barrel flange 44 is positioned at an opposite axial end of the barrel 12 as the end in which a needle would be coupled. Further, the barrel flange 44 is a feature of the barrel 12 that a user grasps with their fingers during the injection process of the syringe 10. The barrel flange 44 extends radially outwards from an outer circumference of the barrel 12, with respect to a central axis of the barrel 12, such that the barrel flange 44 has a greater width and/or diameter than an outer surface of the barrel 12.

When the prefilled syringe 10 is opened and removed from its packaging, as illustrated in FIG. 6A, the plunger 14 extends axially out from the end of the barrel 12 including the barrel flange 44. Further, the conductive ring 18 is separated by a gap (i.e., axially offset) from the barrel flange 44. Once the syringe 10 has been primed and the injection is being performed, as illustrated in FIG. 6B, the plunger 14 axially translates into the barrel 12, and the conductive ring 18 translates axially towards the barrel 12 until the conductive ring 18 is pressed against a surface or face of the barrel flange 44. Specifically, the conductive ring 18 translates axially with the plunger 14 until the conductive ring 18 contacts the barrel flange 44, and then the conductive ring 18 remains in contact with the barrel flange 44 until the injection process is completed. Therefore, as long as the plunger 14 moves axially into the barrel 12, the conductive ring 18 is forced to stay in position on the surface or face of the barrel flange 44 due to the mechanical interference with the barrel flange 44.

Further, during the axial insertion or translation of the plunger 14 into the barrel 12, the sliding contacts 42 of the conductive ring 18 are pressed against and slide along the ground contact 36 and the plurality of contact pads 38, which are visible and/or accessible between the first body portion 22 and the second body portion 24 of the plunger 14. The contact and connection between the sliding contacts 42 and the ground contact 36 and the plurality of contact pads 38 produces electrical/digital signals that are transferred to the BLE chip 34 through digital input lines or other conductive connection paths on or within the printed circuit board 16. The BLE chip 34 gathers the data and is able to analyze the data to distinguish the actual position of the plunger 14 relative to the barrel 12, which indicates the status of the injection process. In other words, the electrical connection between the conductive ring 18 and the printed circuit board 16 can be recorded and analyzed by the BLE chip 34 to indicate how far into the injection process the syringe 10 is, and when the injection process of the syringe 10 is complete and the fluid within the syringe 10 has been fully dispensed. Therefore, when the injection is completed, the plunger 14 includes a specific/distinguishable contact pad 38 on the printed circuit board 16 that identifies the final position of the plunger 14 and a proper completion of the injection cycle. As such, the plurality of contact pads 38 are each configured to produce a unique electrical or digital signal that is transferred to the BLE chip 34. Then the BLE chip 34 can analyze the unique electrical or digital signal to determine how far into the injection process the plunger 14 has completed (e.g. initial/start position, halfway through injection process, injection process completed, etc.).

The BLE chip 34 can further be wirelessly connected to a smartphone or other connected device (e.g., a controller, a computer, cloud networking, etc.) and the BLE chip 34 can be configured to transfer the collected data to the connected device for further storing and analysis. As such, the syringe 10 is configured to produce, collect, and record data related to the status of the injection process, and then the syringe 10 is configured to transfer that data from the syringe 10 to a remotely connected device through a wireless transfer protocol. It is also noted that the conductive ring 18 and the plunger 14 are free to rotate about a central axis of the syringe 10 within the barrel 12 since there are no mechanical constraints preventing such rotation. Specifically, the sliding contacts 42 of the conductive ring 18 are positioned within small gaps on either side of the plunger 14 that are created by the connection of the first body portion 22 and the second body portion 24. This connection facilitates the sliding contacts generally being within or flush with an outer diameter of the plunger 14 that is configured to be positioned within the barrel 12. Additionally, the connected conductive ring 18 and the plunger being axially rotatable about an axis of the plunger 14 prevents issues or damage during assembly, which prevents damage and increases ease of use by the user or patient.

The syringe 10 including the printed circuit board 16 within the plunger 14 and the conductive ring 18 provides a digital readout of the injection process and the position of the plunger 14 relative to the barrel 12, which data is useful in clinical trials as well as overall patient monitoring. In some examples, the plurality of contact pads 38 can be configured such that the syringe 10 can identify six positional sensing positions of the plunger 14 relative to the barrel 12 (i.e., initial start position, 50% injection completed, 70% injection completed, 80% injection completed, 90% injection completed, and 100% injection completed). In other examples, the plurality of contact pads 38 can be configured such that the syringe 10 can identify more or less than six positional sensing positions of the plunger 14 relative to the barrel 12. In any case, the data collected can identify the name of the fluid medication, whether a user/patient has administered the correct dosage of the fluid medication, the time and day the fluid medication was dispensed, and/or the speed or duration in which the fluid medication was dispensed. Each of the aforementioned data points can be utilized to analyze the efficacy of the fluid medication and whether changes are required and/or if the patient is correctly administering the fluid medication as instructed.

The syringe 10 including the plunger 14 with the printed circuit board 16 and the conductive ring 18 is an innovative electromechanical switch sensing architecture that is paired with a low power BLE chip 34, which assembly allows the creation of a smart syringe 10 used to produce and transfer injection progress data of the syringe 10 to an application on a smartphone or other connected device. The miniaturization of electronics and the novel position sensor concept disclosed make the body of the syringe 10 compatible with existing thermoformed packages, and the reduced number of components results in a cheaper bill of material and overall syringe 10, compared to previous smart syringes. In some examples, the data can be collected with a medical grade hub or smartphone, and the measurement of the delivered dose can be encrypted for data safety. For at least those reasons, it is clear that the syringe 10 of the present disclosure is advantageous over previous smart syringes.

Additionally, the syringe 10 of the present disclosure provides a cost-effective method to monitor and wirelessly report the position of the plunger 14 of the syringe 10 during the injection process. The plurality of contact pads 38 leveraging a side plating on the printed circuit board 16, and the conductive ring 18 sliding along the plunger 14 is configured to capture the position of the plunger 14 as it is moving axially into the barrel 12 during the injection. This position of the plunger 14 is monitored and analyzed by the BLE chip 34 (i.e., microcontroller) as a digital input change, and a newly computed status is sent via the BLE chip 34 to the smartphone or other connected device on a periodic notification period. Therefore, the smart syringe 10 of the present disclosure can be utilized to automatically gather data relating to the injection process which can be utilized for clinical trials and to enable therapy adherence monitoring.

As will be appreciated by a person having ordinary skill in the art, the smart syringe 10 of the present disclosure can include integrated security protocols to ensure data protection during the collection and transfer of data. The smart syringe 10 can include BLE connectivity to smartphones and other gateways to enable measurements of the delivered dose of medication, and to enable therapy adherence monitoring. Further, the smart syringe 10 can be the same size and dimensions as existing syringes to aid in the ease of use and familiarity of the product, for example the smart syringe 10 can be a 1 mL prefilled syringe in some embodiments. To this point, the smart syringe 10 can be compatible with existing syringe packages to aid in packaging and delivery of the device. Finally, the smart syringe 10 can be produced with an optimized bill of material and a long shelf life (e.g. 4+ years) to create a relatively cheap smart syringe 10 that is also disposable. In some examples, the smart syringe 10 can be produced from eco-friendly resins (e.g. polycarbonates, polypropylene, etc.) such that the smart syringe 10 is at least partially recyclable. The smart syringe 10 of the present application provides many advantages over previous smart syringes, as will be appreciated by those having skill in the art.

Having thus described the present embodiments in detail, it is to be appreciated and will be apparent to those skilled in the art that many physical changes, only a few of which are exemplified in the detailed description of the disclosure, could be made without altering the inventive concepts and principles embodied therein.

It is also to be appreciated that numerous embodiments incorporating only part of the preferred embodiment are possible which do not alter, with respect to those parts, the inventive concepts and principles embodied therein. The present embodiment and optional configurations are therefore to be considered in all respects as exemplary and/or illustrative and not restrictive, the scope of the disclosure being indicated by the appended claims rather than by the foregoing description.

### Log of Reference Numerals

- 10: Syringe
- 12: Barrel
- 14: Plunger
- 16: Printed Circuit Board
- 16A: First End
- 16B: Second End
- 18: Conductive Ring
- 20: Plunger End
- 22: First Body Portion
- 22A: First End
- 22B: Second End
- 24: Second Body Portion
- 24A: First End
- 24B: Second End
- 26: Pin
- 28: Aperture
- 30: Battery Connector
- 32: Battery
- 34: BLE Chip
- 36: Ground Contact
- 38: Contact Pads
- 40: Conductive Ring Body
- 42: Sliding Contacts
- 44: Barrel Flange
- CA: Central Axis

## Claims

1. A syringe (10) comprising:
a barrel (12) including a barrel flange (44) positioned at an axial end of the barrel (12);
a plunger (14) axially translatable within the barrel (12) along a central axis of the plunger (14), the plunger (14) including a first body portion (22) coupled to a second body portion (24);
a printed circuit board (16) coupled to and positioned between the first body portion (22) and the second body portion (24), the printed circuit board (16) comprising:
a ground contact (36) disposed on a first lateral side of the printed circuit (16);
a plurality of contact pads (38) disposed on a second lateral side of the printed circuit board (16); and
a microcontroller (34) coupled to a surface of the printed circuit board (16); and
a conductive ring (18) disposed about an outer circumference of the plunger (14), the conductive ring (18) including sliding contacts (42) configured to engage the ground contact (36) and the plurality of contact pads (38) of the printed circuit board (16);
wherein the microcontroller (34) is configured to detect a change in conductivity to identify a position of the plunger (14) relative to the barrel (12).

2. The syringe (10) of claim 1, wherein the first body portion (22) and the second body portion (24) are
coupled together through a snap-fit connection; or
a first half portion and a second half portion of the plunger (14), respectively.

3. The syringe (10) of claim 1, wherein the second body portion (24) includes
a pin (26) positioned on a radially inner surface of the second body portion (24), relative to the central axis of the plunger (14); or
a pin (26) positioned on a radially inner surface of the second body portion (24), relative to the central axis of the plunger (14); wherein the printed circuit board (16) includes an aperture (28) extending through the printed circuit board (16), the aperture (28) being configured to accept the pin (16) of the second body portion (24) for positioning of the printed circuit board (16) relative to the second body portion (24).

4. The syringe (10) of claim 1, wherein the printed circuit board includes
a battery connector (30) disposed at an end of the printed circuit board (16), the battery connector (30) being configured for accepting and securing a battery (32) of the syringe; or
a battery connector (30) disposed at an end of the printed circuit board (16), the battery connector (30) being configured for accepting and securing a battery (32) of the syringe; wherein the battery (32) is a coin cell battery.

5. The syringe (10) of claim 1, wherein the ground contact (36) of the printed circuit board (16) is a continuous ground contact that extends from a first end (16A) of the printed circuit board (16) to a second end (16B) of the printed circuit board (16).

6. The syringe (10) of claim 1, wherein the plurality of contact pads (38) are a discontinuous group of individual contact pads that extend from a first end (16A) of the printed circuit board (16) to a second end (16B) of the printed circuit board (16).

7. The syringe (10) of claim 6, wherein the discontinuous group of individual contact pads (38) includes a nonconductive space positioned between each adjacent contact pad of the plurality of contact pads (38).

8. The syringe (10) of claim 1, wherein the microcontroller (34) is a Bluetooth low energy chip that is configured to wirelessly transmit data to a device remote from the syringe.

9. The syringe (10) of claim 1, wherein the surface of the printed circuit board (16) the microcontroller (34) is coupled to is perpendicular to the first lateral side and the second lateral side of the printed circuit board (16).

10. The syringe (10) of claim 1, wherein the first lateral side of the printed circuit board (16) is parallel with the second lateral side of the printed circuit board (16).

11. The syringe (10) of claim 1, wherein the syringe is a pre-filled disposable syringe.

12. The syringe (10) of claim 1, wherein the conductive ring (18) includes a circular shaped conductive ring body (40) and a first sliding contact and a second sliding contact (42) each extending axially away from the conductive ring body (40).

13. The syringe (10) of claim 12, wherein the first sliding contact is configured to engage the ground contact (36) of the printed circuit board (16), and the second sliding contact (42) is configured to engage the plurality of contact pads (38) of the printed circuit board (16).

14. The syringe (10) of claim 1, wherein the conductive ring (18) includes an inner diameter greater than an outer diameter of a portion of the plunger (14) configured to axially translate into the barrel (12).

15. The syringe (10) of claim 1, wherein the conductive ring (18)
is axially translatable along an outer surface of the plunger (14) during an axial translation of the plunger (14) into the barrel (12); or
is axially translatable along an outer surface of the plunger (14) during an axial translation of the plunger (14) into the barrel (12); wherein during the axial translation of the plunger (14) into the barrel (12) the conductive ring (18) contacts the barrel flange (44), preventing axial translation of the conductive ring (18) relative to the barrel (12); or
is axially translatable along an outer surface of the plunger (14) during an axial translation of the plunger (14) into the barrel (12); wherein before the axial translation of the plunger (14) into the barrel (12), the conductive ring is axially offset from the barrel flange (44).

## Patentansprüche

1. Spritze (10), umfassend:
einen Zylinder (12), der einen Zylinderflansch (44) beinhaltet, der an einem axialen Ende des Zylinders (12) positioniert ist;
einen Kolben (14), der innerhalb des Zylinders (12) entlang einer Mittelachse des Kolbens (14) axial verschiebbar ist, wobei der Kolben (14) einen ersten Körperabschnitt (22) beinhaltet, der mit einem zweiten Körperabschnitt (24) gekoppelt ist;
eine Leiterplatte (16), die mit dem ersten Körperabschnitt (22) und dem zweiten Körperabschnitt (24) gekoppelt und zwischen diesen positioniert ist, wobei die Leiterplatte (16) Folgendes umfasst:
einen Massekontakt (36), der an einer ersten Seitenfläche der Leiterplatte (16) angeordnet ist;
eine Mehrzahl von Kontaktflächen (38), die an einer zweiten Seitenfläche der Leiterplatte (16) angeordnet sind; und
einen Mikrocontroller (34), der mit einer Oberfläche der Leiterplatte (16) gekoppelt ist; und
einen leitfähigen Ring (18), der um einen Außenumfang des Kolbens (14) herum angeordnet ist, wobei der leitfähige Ring (18) Schleifkontakte (42) beinhaltet, die dazu ausgebildet sind, mit dem Massekontakt (36) und der Mehrzahl von Kontaktflächen (38) der Leiterplatte (16) in Eingriff zu treten;
wobei der Mikrocontroller (34) dazu ausgebildet ist, eine Änderung der Leitfähigkeit zu erfassen, um eine Position des Kolbens (14) relativ zu dem Zylinder (12) zu identifizieren.

2. Spritze (10) nach Anspruch 1, wobei der erste Körperabschnitt (22) und der zweite Körperabschnitt (24) durch eine Schnappverbindung miteinander gekoppelt sind; oder
jeweils ein erster Halbabschnitt und ein zweiter Halbabschnitt des Kolbens (14) sind.

3. Spritze (10) nach Anspruch 1, wobei der zweite Körperabschnitt (24) Folgendes beinhaltet
einen Stift (26), der an einer radial inneren Oberfläche des zweiten Körperabschnitts (24) relativ zu der Mittelachse des Kolbens (14) positioniert ist; oder
einen Stift (26), der an einer radial inneren Oberfläche des zweiten Körperabschnitts (24) relativ zu der Mittelachse des Kolbens (14) positioniert ist; wobei die Leiterplatte (16) eine Öffnung (28) beinhaltet, die sich durch die Leiterplatte (16) hindurch erstreckt, wobei die Öffnung (28) dazu ausgebildet ist, den Stift (26) des zweiten Körperabschnitts (24) zur Positionierung der Leiterplatte (16) relativ zu dem zweiten Körperabschnitt (24) aufzunehmen.

4. Spritze (10) nach Anspruch 1, wobei die Leiterplatte Folgendes beinhaltet
einen Batterieanschluss (30), der an einem Ende der Leiterplatte (16) angeordnet ist, wobei der Batterieanschluss (30) dazu ausgebildet ist, eine Batterie (32) der Spritze aufzunehmen und zu sichern; oder
einen Batterieanschluss (30), der an einem Ende der Leiterplatte (16) angeordnet ist, wobei der Batterieanschluss (30) dazu ausgebildet ist, eine Batterie (32) der Spritze aufzunehmen und zu sichern; wobei die Batterie (32) eine Knopfzellenbatterie ist.

5. Spritze (10) nach Anspruch 1, wobei der Massekontakt (36) der Leiterplatte (16) ein kontinuierlicher Massekontakt ist, der sich von einem ersten Ende (16A) der Leiterplatte (16) zu einem zweiten Ende (16B) der Leiterplatte (16) erstreckt.

6. Spritze (10) nach Anspruch 1, wobei die Mehrzahl von Kontaktflächen (38) eine diskontinuierliche Gruppe einzelner Kontaktflächen ist, die sich von einem ersten Ende (16A) der Leiterplatte (16) zu einem zweiten Ende (16B) der Leiterplatte (16) erstrecken.

7. Spritze (10) nach Anspruch 6, wobei die diskontinuierliche Gruppe einzelner Kontaktflächen (38) einen nicht leitfähigen Zwischenraum beinhaltet, der zwischen jeder benachbarten Kontaktfläche der Mehrzahl von Kontaktflächen (38) positioniert ist.

8. Spritze (10) nach Anspruch 1, wobei der Mikrocontroller (34) ein Bluetooth-Low-Energy-Chip ist, der dazu ausgebildet ist, Daten drahtlos an eine von der Spritze entfernte Vorrichtung zu übertragen.

9. Spritze (10) nach Anspruch 1, wobei diejenige Oberfläche der Leiterplatte (16), mit der der Mikrocontroller (34) gekoppelt ist, senkrecht zu der ersten Seitenfläche und der zweiten Seitenfläche der Leiterplatte (16) ist.

10. Spritze (10) nach Anspruch 1, wobei die erste Seitenfläche der Leiterplatte (16) parallel zu der zweiten Seitenfläche der Leiterplatte (16) ist.

11. Spritze (10) nach Anspruch 1, wobei die Spritze eine vorgefüllte Einwegspritze ist.

12. Spritze (10) nach Anspruch 1, wobei der leitfähige Ring (18) einen kreisförmigen leitfähigen Ringkörper (40) sowie einen ersten Schleifkontakt und einen zweiten Schleifkontakt (42) beinhaltet, die sich jeweils axial von dem leitfähigen Ringkörper (40) weg erstrecken.

13. Spritze (10) nach Anspruch 12, wobei der erste Schleifkontakt dazu ausgebildet ist, mit dem Massekontakt (36) der Leiterplatte (16) in Eingriff zu treten, und der zweite Schleifkontakt (42) dazu ausgebildet ist, mit der Mehrzahl von Kontaktflächen (38) der Leiterplatte (16) in Eingriff zu treten.

14. Spritze (10) nach Anspruch 1, wobei der leitfähige Ring (18) einen Innendurchmesser beinhaltet, der größer ist als ein Außendurchmesser eines Abschnitts des Kolbens (14), wobei dieser Abschnitt dazu ausgebildet ist, sich axial in den Zylinder (12) zu verschieben.

15. Spritze (10) nach Anspruch 1, wobei der leitfähige Ring (18)
entlang einer äußeren Oberfläche des Kolbens (14) während einer axialen Verschiebung des Kolbens (14) in den Zylinder (12) axial verschiebbar ist; oder
entlang einer äußeren Oberfläche des Kolbens (14) während einer axialen Verschiebung des Kolbens (14) in den Zylinder (12) axial verschiebbar ist; wobei der leitfähige Ring (18) während der axialen Verschiebung des Kolbens (14) in den Zylinder (12) den Zylinderflansch (44) berührt, wodurch eine axiale Verschiebung des leitfähigen Rings (18) relativ zu dem Zylinder (12) verhindert wird; oder
entlang einer äußeren Oberfläche des Kolbens (14) während einer axialen Verschiebung des Kolbens (14) in den Zylinder (12) axial verschiebbar ist; wobei der leitfähige Ring vor der axialen Verschiebung des Kolbens (14) in den Zylinder (12) axial gegenüber dem Zylinderflansch (44) versetzt ist.

## Revendications

1. Seringue (10) comprenant :
un cylindre (12) comportant un collet de cylindre (44) positionné à une extrémité axiale du cylindre (12) ;
un piston (14) pouvant se déplacer axialement à l'intérieur du cylindre (12) le long d'un axe central du piston (14), le piston (14) comportant une première partie de corps (22) couplée à une seconde partie de corps (24) ;
une carte de circuit imprimé (16) couplée à la première partie de corps (22) et à la seconde partie de corps (24) et positionnée entre celles-ci, la carte de circuit imprimé (16) comprenant :
un contact de masse (36) disposé sur un premier côté latéral de la carte de circuit imprimé (16) ;
une pluralité de plots de contact (38) disposés sur un second côté latéral de la carte de circuit imprimé (16) ; et
un microcontrôleur (34) couplé à une surface de la carte de circuit imprimé (16) ; et
une bague conductrice (18) disposée autour d'une circonférence extérieure du piston (14), la bague conductrice (18) comportant des contacts frottants (42) configurés pour venir en prise avec le contact de masse (36) et la pluralité de plots de contact (38) de la carte de circuit imprimé (16) ;
dans laquelle le microcontrôleur (34) est configuré pour détecter une variation de conductivité afin d'identifier une position du piston (14) par rapport au cylindre (12).

2. Seringue (10) selon la revendication 1, dans laquelle la première partie de corps (22) et la seconde partie de corps (24) sont couplées l'une à l'autre par un raccordement par encliquetage ; ou
sont, respectivement, une première demi-partie et une seconde demi-partie du piston (14).

3. Seringue (10) selon la revendication 1, dans laquelle la seconde partie de corps (24) comporte
une broche (26) positionnée sur une surface radialement intérieure de la seconde partie de corps (24), par rapport à l'axe central du piston (14) ; ou
une broche (26) positionnée sur une surface radialement intérieure de la seconde partie de corps (24), par rapport à l'axe central du piston (14) ; dans laquelle la carte de circuit imprimé (16) comporte une ouverture (28) s'étendant à travers la carte de circuit imprimé (16), l'ouverture (28) étant configurée pour recevoir la broche (16) de la seconde partie de corps (24) en vue du positionnement de la carte de circuit imprimé (16) par rapport à la seconde partie de corps (24).

4. Seringue (10) selon la revendication 1, dans laquelle la carte de circuit imprimé comporte
un connecteur de pile (30) disposé à une extrémité de la carte de circuit imprimé (16), le connecteur de pile (30) étant configuré pour recevoir et assujettir une pile (32) de la seringue ; ou
un connecteur de pile (30) disposé à une extrémité de la carte de circuit imprimé (16), le connecteur de pile (30) étant configuré pour recevoir et assujettir une pile (32) de la seringue ; dans laquelle la pile (32) est une pile bouton.

5. Seringue (10) selon la revendication 1, dans laquelle le contact de masse (36) de la carte de circuit imprimé (16) est un contact de masse continu qui s'étend d'une première extrémité (16A) de la carte de circuit imprimé (16) à une seconde extrémité (16B) de la carte de circuit imprimé (16).

6. Seringue (10) selon la revendication 1, dans laquelle la pluralité de plots de contact (38) est un groupe discontinu de plots de contact individuels qui s'étendent d'une première extrémité (16A) de la carte de circuit imprimé (16) à une seconde extrémité (16B) de la carte de circuit imprimé (16).

7. Seringue (10) selon la revendication 6, dans laquelle le groupe discontinu de plots de contact individuels (38) comporte un espace non conducteur positionné entre chaque plot de contact adjacent de la pluralité de plots de contact (38).

8. Seringue (10) selon la revendication 1, dans laquelle le microcontrôleur (34) est une puce Bluetooth à basse consommation qui est configurée pour transmettre sans fil des données à un dispositif distant de la seringue.

9. Seringue (10) selon la revendication 1, dans laquelle la surface de la carte de circuit imprimé (16) à laquelle le microcontrôleur (34) est couplé est perpendiculaire au premier côté latéral et au second côté latéral de la carte de circuit imprimé (16).

10. Seringue (10) selon la revendication 1, dans laquelle le premier côté latéral de la carte de circuit imprimé (16) est parallèle au second côté latéral de la carte de circuit imprimé (16).

11. Seringue (10) selon la revendication 1, dans laquelle la seringue est une seringue jetable préremplie.

12. Seringue (10) selon la revendication 1, dans laquelle la bague conductrice (18) comporte un corps de bague conductrice de forme circulaire (40) ainsi qu'un premier contact frottant et un second contact frottant (42) s'étendant chacun axialement à distance du corps de bague conductrice (40).

13. Seringue (10) selon la revendication 12, dans laquelle le premier contact frottant est configuré pour venir en prise avec le contact de masse (36) de la carte de circuit imprimé (16), et le second contact frottant (42) est configuré pour venir en prise avec la pluralité de plots de contact (38) de la carte de circuit imprimé (16).

14. Seringue (10) selon la revendication 1, dans laquelle la bague conductrice (18) comporte un diamètre intérieur supérieur à un diamètre extérieur d'une portion du piston (14) configurée pour effectuer une translation axiale dans le cylindre (12).

15. Seringue (10) selon la revendication 1, dans laquelle la bague conductrice (18)
peut se déplacer axialement le long d'une surface extérieure du piston (14) au cours d'une translation axiale du piston (14) dans le cylindre (12) ; ou
peut se déplacer axialement le long d'une surface extérieure du piston (14) au cours d'une translation axiale du piston (14) dans le cylindre (12) ; dans laquelle, au cours de la translation axiale du piston (14) dans le cylindre (12), la bague conductrice (18) entre en contact avec le collet de cylindre (44), empêchant la translation axiale de la bague conductrice (18) par rapport au cylindre (12) ; ou
peut se déplacer axialement le long d'une surface extérieure du piston (14) au cours d'une translation axiale du piston (14) dans le cylindre (12) ; dans laquelle, avant la translation axiale du piston (14) dans le cylindre (12), la bague conductrice est décalée axialement par rapport au collet de cylindre (44).
